# EUROPEAN PATENT SPECIFICATION

(11) **EP 0 417 781 B1**
(45) Date of publication and mention of the grant of the patent: **08.03.1995**
(21) Application number: 90117650.3
(22) Date of filing: 13.09.1990
(51) Int. Cl.: A61M 25/00, A61M 25/10, A61B 5/02

(54) **Cardiac output measuring catheter**
Katheter zur Messung des Herzausstosses
Cathéter pour mesurer le rendement du coeur

(30) Priority: 14.09.1989 JP 238663/89
(43) Date of publication of application: 20.03.1991
(73) Proprietor: TERUMO KABUSHIKI KAISHA, Tokyo 151 (JP)
(72) Inventor: Yoshikoshi, Akiko, c/o Terumo K.K., Ashigarakami-gun, Kanagawa-ken (JP); Tsuchida, Kouji, c/o Terumo K.K., Ashigarakami-gun, Kanagawa-ken (JP)
(74) Representative: Casalonga, Axel

(56) References cited:
- EP-A- 0 246 176
- EP-A- 0 303 757
- US-A- 4 721 115
- US-A- 4 841 981

## Description

This invention relates to a cardiac output measuring catheter adapted to be connected to a cardiac output measuring apparatus used for heart function examination.

For heart function examination, a cardiac output is measured by a right heart catheter technique which typically relies on thermodilution. In the right heart catheter technique, the catheter is introduced from a jugular vein, femoral vein, cubital vein or the like and advanced through the superior or inferior vena cava, right atrium and right ventricle until its distal end reaches the pulmonary artery.

The catheter is provided with an injection port and a thermistor as a temperature sensing element such that the injection port is located in the right atrium and the thermistor is located in the pulmonary artery when the catheter is set in place. The injection port through which a fluid having a higher or lower temperature than the blood is introduced is located proximal of the thermistor. The temperature of the fluid as discharged is diffused and diluted in the right atrium and right ventricle. The diluted temperature is detected by the thermistor located in the pulmonary artery. A cardiac output is determined from a change with time of the detected temperature, that is, a dilution curve according to Stewart-Hamilton method.

The cardiac output measurement by the thermodilution method is, however, intermittent and not applicable to continuous cardiac output measurement. When it is desired to take a plurality of measurements, the total amount of fluid introduced is increased, which imposes an increased burden to the patient. Repetition of such operations can increase the risk of infection.

U.S. Patent No. 4,841,981 discloses a catheter for the continuous measurement of a cardiac output. This catheter includes temperature sensors for thermodilution measurement of a cardiac output and for measurement of a velocity of blood flow. It provides continuous measurement of blood flow velocity, from which a cardiac output is continuously calculated.

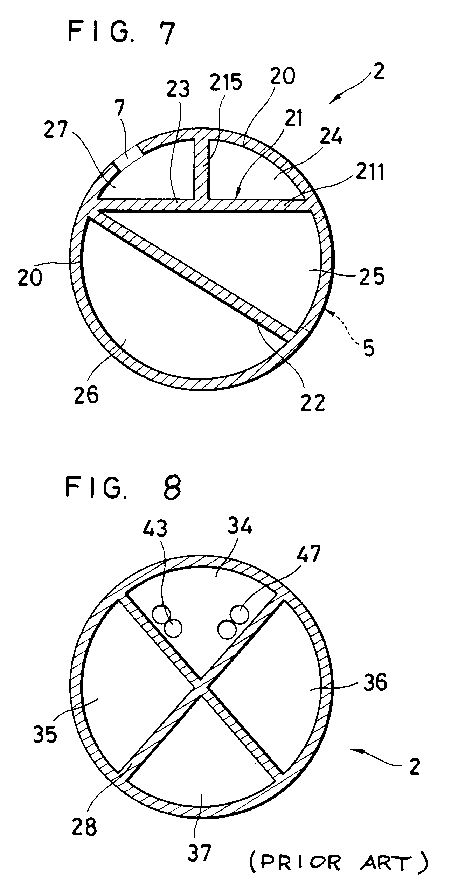

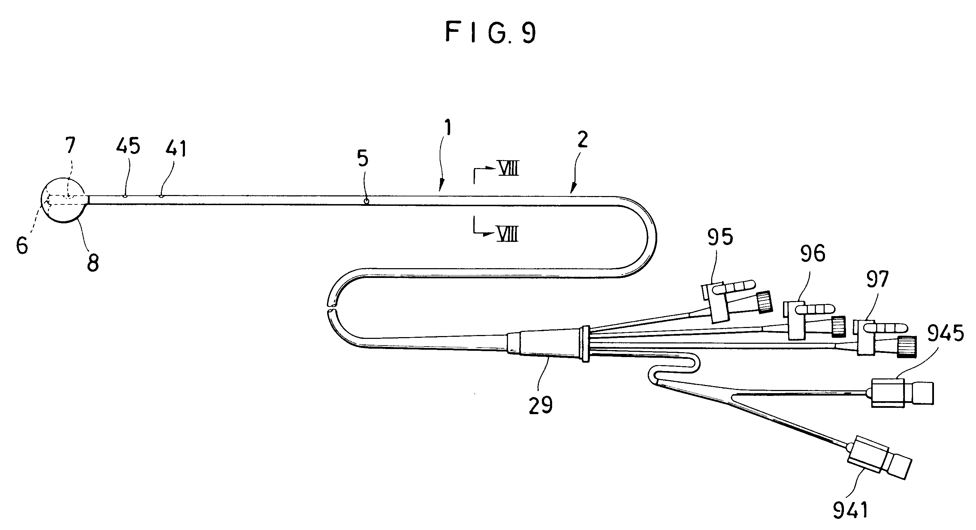

A typical arrangement of the catheter is shown in FIGS. 8 and 9. The catheter generally designated at 1 includes an elongated catheter tube 2 defining four lumens therein and having distal and proximal ends. The catheter 1 includes a pressure port 6 at the distal end of the catheter tube 2 and a balloon 8 of soft elastomer attached thereto so as to entirely surround the catheter tube over a distance of several mm from its distal end. The catheter tube 2 is provided with a side port 7 formed in the tube side wall surrounded by the balloon 8 for passage of a gas such as carbon dioxide for inflating and deflating the balloon, a second thermistor 45 spaced 10 to 20 mm apart from the distal end, a first thermistor 41 spaced 10 to 15 mm apart from the second thermistor 45 toward the proximal end, and an injection port 5 disposed proximal of the thermistors 41 and 45 and spaced 12 to 40 cm apart from the distal end.

Since this catheter is for arterial use, that is, adapted to be left in the pulmonary artery by inserting from a superior or inferior limb vein, thermistors 41 and 45 are provided on a catheter distal portion which is located downstream of the injection port 5 in the direction of blood flow. If the catheter is for venous use, that is, adapted to be left in the pulmonary vein, thermistors 41 and 45 should be provided on a catheter proximal portion which is located downstream of the injection port 5 in the blood flow direction.

The pressure port 6, balloon side port 7, thermistors 41 and 45, and injection port 5 are in communication with four independent lumens, respectively. More particularly, the pressure port 6 is in communication with a lumen 36 for measuring the external pressure such as pulmonary arterial pressure, the balloon side port 7 in communication with a balloon lumen 37, the thermistors 41 and 45 in communication with a lumen 34, and the injection port 5 in communication with a lumen 35 for injecting indicator fluid.

At the proximal end 29 of the catheter tube 2, the thermistor lumen 34 is coupled to connectors 941 and 945 through a bifurcated section of tubing such that lead wires 43 and 47 extending from the thermistors 41 and 45 are electrically connected to the connectors 941 and 945 through the thermistor lumen 34 and the section of tubing. Also, the pressure measuring lumen 36, injecting lumen 35 and balloon lumen 37 are coupled to connectors 96, 95 and 97 through sections of tubing, respectively.

The first thermistor 41 serving as a temperature sensor is for thermodilution measurement of a cardiac output whereas the second thermistor 45 is a thermistor of direct or indirect heating type which is adapted to be heated by electricity conduction or by another heater and produces a heating temperature signal representative of its own temperature, thereby sensing a blood flow velocity.

The four lumens 34, 35, 36 and 37 are defined in the interior bore of the catheter tube 2 by dividing the bore by a crisscross partition 28 into four sectors disposed about the center as shown in FIG. 9. The measuring lumen 36 is disposed adjacent the thermistor lumen 34.

On clinical application, the cardiac output measuring catheter, which is adapted for continuous measurement, is typically used in measurement for a long period of time, during which continuous fluid infusion is often needed. More particularly, it is often necessary to continuously infuse a suitable fluid through the pressure port 6 at the catheter distal end for pressure measurement. If such fluid is passed through the pressure measuring lumen 36 which is disposed adjacent the thermistor lumen 34, then the thermistors 41 and 45 are thermally affected by the fluid. In principle, the second thermistor 45 which is of the heating type is generally heated by conduction of constant current and senses its own temperature at the same time. Blood flow velocity is determined from the temperatures the first and second thermistors 41 and 45 sense and the heating current. If the temperature in the thermistor lumen 34 is substantially affected by the temperature of the fluid passing through the pressure measuring lumen 36, there occurs an inconvenience that the heating temperature signal, blood temperature signal and the value of conducting current can change, failing in accurate detection of blood flow velocity. Such thermal influence will also occur when only the first thermistor 41 for measuring a cardiac output is used.

Furthermore, the thermistor lumen 34 is disposed contiguous to the measuring lumen 36 in the above-cited publication. With the partition structure disclosed therein and illustrated in FIG. 9, even if the pressure measuring lumen is changed to lumen 37 to separate the thermistor lumen 34 from the measuring lumen (37), there still remains a risk of failing to provide accurate measurement of blood flow velocity because the partition sections are contiguous.

EP-A-0 246 176 discloses a catheter where the sensing means lumen and the indicator fluid lumen are separated by a partition wall comprising one or several air openings, therethrough. It is, however, neither disclosed nor tought that this insulating structure can be used for a fluid.

Therefore, an object of the present invention is to provide a novel and improved cardiac output measuring catheter which can measure both a cardiac output and a blood flow velocity while allowing infusion of a fluid, typically a transfusion fluid into the associated blood vessel through a distal end opening of the catheter without adversely affecting the cardiac output measurement.

As claimed, the catheter of the invention for measuring a cardiac output, comprises a catheter tube having a wall defining a longitudinal bore therein and having a distal end and a proximal end and an opening near the distal end and a balloon attached to said catheter tube adjacent its distal end.

Partition means are provided in the tube for dividing the bore into a first lumen for installing therein temperature sensing means disposed on a proximal side with respect to said opening, a second lumen in communication with the opening and at least two third lumens.

According to the invention, a first partition defining said first lumen is disposed so as not to intersect a second partition defining said second lumen, whereby said first and second lumens are separated by an injecting lumen having an injection port for allowing an indicator fluid to pass therethrough and exit through the injection port, said injection port being located on a proximal side with respect to the temperature sensing means and by a balloon lumen in communication with said balloon.

Preferably, the partition means includes a third partition for defining said two third lumens. The third partition may extend between opposed positions along the tube wall. It may also extend between the first and second partitions.

Preferably, the temperature sensing means includes a thermistor for thermodilution measurement of a cardiac output and a heating thermistor for measurement of blood flow velocity.

Preferably, the second lumen is in fluid communication with the exterior through the opening for allowing measurement of the external pressure.

Preferably, the third lumen includes a lumen having an injection port for allowing an indicator fluid for thermodilution measurement of a cardiac output to pass therethrough and exit through the injection port, the injection port being located on a proximal side with respect to the temperature sensing means.

The catheter may further include a balloon attached to the catheter tube adjacent its distal end, and the third lumen includes a lumen in fluid communication with the balloon.

The invention will now be more precisely described on the basis of some non limitative examples illustrated on the appended drawings on which:
FIG. 1 is an axial cross section of a distal portion of a cardiac output measuring catheter according to one embodiment of the invention.
FIG. 2 is an enlarged transverse cross section of the catheter taken along lines II-II in FIG. 1.
FIGS. 3 to 7 are enlarged transverse cross sections similar to FIG. 2 of different catheter embodiments of the invention.
FIG. 8 is an enlarged transverse cross section of the catheter taken along lines VIII-VIII in FIG. 9.
FIG. 9 is a plan view of the outline of a prior art cardiac output measuring catheter.

The outline of the cardiac output measuring catheter according to the present invention is the same as the prior art cardiac output measuring catheter disclosed in US-A-4 841 981 and shown in FIG. 9. Except the partition configuration in the catheter tube bore, the structure and function of the present catheter are identical with the prior art catheter. If desired, the cardiac output measuring catheter of the present invention may be of the construction having only the first thermistor 41 installed in the thermistor lumen for measuring a cardiac output.

Basically, the cardiac output measuring catheter of the present invention includes an elongated catheter tube having a distal end and a proximal end and defining at least three (first, second and third) longitudinally extending lumens in its bore. The catheter includes at least one temperature sensing element typically in the form of a thermistor.

The first lumen is at least one lumen which is assigned as a thermistor lumen, that is, a lumen having a thermistor installed therein. Usually, a single lumen is commonly used for the first and second thermistors 41 and 45 with their lead wires although a plurality of thermistors may be independently accommodated in separate lumens.

The second lumen is at least one lumen having an opening or port located distal of the thermistor installed in the first lumen. The second lumen is typically a lumen having a pressure port for measuring the external pressure, typically the pressure of pulmonary artery when the catheter is intended for use with its distal end left in the pulmonary artery. Another lumen having a port for infusing a fluid may be additionally provided. In the case of a catheter whose distal end is to be left in the pulmonary vein in which a lumen for injecting an indicator fluid has an injection port located distal of the thermistor, the second lumen includes this indicator fluid injecting lumen in addition to the pressure measuring lumen.

The third lumen defined in the bore of the catheter tube should have no or negligible thermal influence on the thermistor(s) and is typically a lumen for injecting an indicator fluid in the case of a catheter whose distal end is to be left in the pulmonary artery. Since an injection port is located proximal of the thermistor in this case, the portion of the indicator injecting lumen that extends from the injection port toward the distal end is blocked with a resin filling at a position proximal of the thermistor(s) to prevent the fluid from reaching the thermistor installed site, thereby eliminating any thermal influence of the indicator fluid on the thermistor(s) as well as any thermal influence of the self-heating thermistor on the indicator fluid.

If the indicator injecting lumen is not blocked and the fluid is thus permitted to flow through the indicator injecting lumen to the distal portion where the thermistor is installed, then this lumen is considered to belong to the second lumen.

The catheter of the invention need not necessarily have a balloon. Provision of a balloon lumen is preferable because a fluid to be introduced into the balloon therethrough, typically a gas such as carbon dioxide has a thermal insulation function, reducing thermal influence. Also included in the third lumen is a lumen for infusing a medicament fluid.

Three or more, typically four or five lumens as mentioned above are defined in the bore of the catheter by providing suitable partition means for dividing the bore in a transverse cross section.

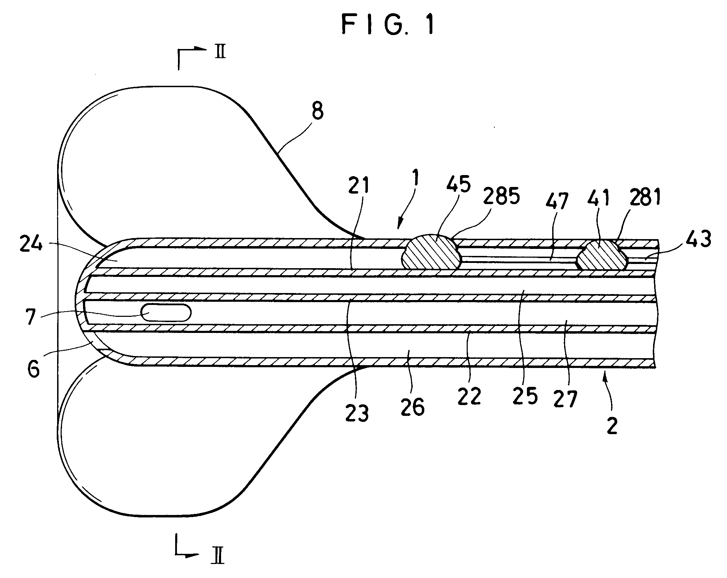

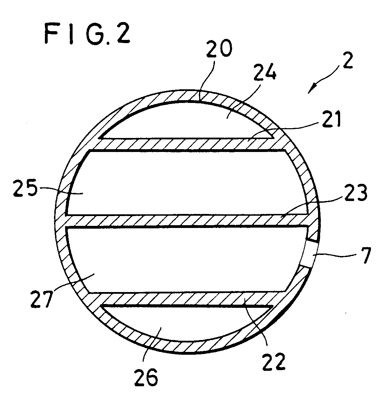

Referring to FIGS. 1 and 2, there is illustrated a distal portion of a catheter according to one embodiment of the invention. The catheter 1 has a catheter tube 2 defining a longitudinally extending bore therein. The tube is provided with partition means including a first partition 21 for defining a thermistor lumen 24 as the first lumen with an arcuate portion of the tube wall 20 and a second partition 22 for defining a pressure measuring lumen 26 as the second lumen with another arcuate portion of the tube wall 20. The first and second partitions 21 and 22 are separate or independent from each other. Differently stated, the first partition 21 does not intersect the second partition 22.

More particularly, the bore of the catheter tube 2 is partitioned into four sections by three longitudinally extending, generally parallel, transversely spaced apart plate-shaped partitions 21, 22 and 23 each extending chordally between opposed positions along the tube wall 20 as shown in FIGS. 1 and 2.

The first partition 21 and an arcuate portion of the tube wall 20 define the thermistor lumen 24 as the first lumen near the bore periphery. The thermistor lumen 24 includes side ports 281 and 285 formed throughout the tube wall 20 and having first and second thermistors 41 and 45 mounted therein, respectively. Lead wires 43 and 47 from the thermistors 41 and 45 extend through the thermistor lumen 24 toward the proximal end. It is to be understood that the first thermistor 41 is a thermistor for thermodilution measurement of a cardiac output and the second thermistor 45 is a thermistor of direct or indirect heating type, preferably self-heating type for measuring a blood flow velocity, respectively, as previously described. For the construction and function of these thermistors, reference should be made to US-A-4 841 981.

The second partition 22 which is approximately parallel and diametrically opposite to the first partition 21 is formed in the bore. The second partition 22 and an arcuate portion of the tube wall 20 define the pressure measuring lumen 26 as the second lumen near the bore periphery. The pressure measuring lumen 26 is in communication with a pressure port in the form of an opening 6 in the distal end wall of the tube 2 so that the external pressure can be measured by way of this lumen 26. Alternatively, a transfusion fluid may be continuously administered through this lumen 26.

The central partition 23 defines an indicator fluid injecting lumen 25 and a balloon lumen 27 with the first and second partitions 21 and 22, respectively. The balloon lumen 27 is in communication with a port 7 in the tube side wall and allows for passage of a gas to and from the balloon 8 for inflation and deflation. The indicator fluid injecting lumen 25 is in communication with the injection port 5 shown in FIG. 9. It is to be noted that the portion of the indicator fluid injecting lumen 25 which extends from the injection port 5 toward the distal end is blocked with a resin plug such as polyvinyl chloride through not shown in the figures. A fluid for cardiac output measurement is discharged through the port 5 and does not internally flow in proximity to the thermistors 41 and 45.

Since the first and second partitions 21 and 22 for defining the thermistor lumen 24 as the first lumen and the pressure measuring lumen 26 as the second lumen are provided separate, any fluid, especially a transfusion fluid introduced into the pressure measuring lumen 26 gives no or little thermal influence on the thermistors 41 and 45, eliminating any disturbance on the measurement of a blood flow velocity.

In the illustrated embodiment, the balloon lumen 27 between the thermistor lumen 24 and the pressure measuring lumen 26 receives a gas for inflation and deflation and a distal portion of the indicator fluid injecting lumen 25 which is blocked with a plug for preventing further entry of the indicator fluid is filled with air or suitable gas. Thermal insulation of these filling gases enhances the benefits of the invention.

Since the thermistors 41, 45 and the injection port 5 are preferably located at a relatively circumferentially close distance on the tube periphery, the indicator fluid injecting lumen 25 is preferably disposed contiguous to the thermistor lumen 24.

If it is desired to introduce a fluid to a distal portion of the indicator fluid injecting lumen 25 or if a catheter is intended for use with its distal end left in the pulmonary vein, the indicator fluid injecting lumen 25 and the balloon lumen 27 are reversed from the illustrated arrangement. Then the partition 23 functions as a second partition with the benefits of the invention because the partitions 21 and 23 are separate from each other.

In a typical example, the catheter tube 2 has an inner diameter of about 0.8 to about 2.5 mm. The first and second partitions are generally spaced a distance of about 0.4 to about 1.5 mm.

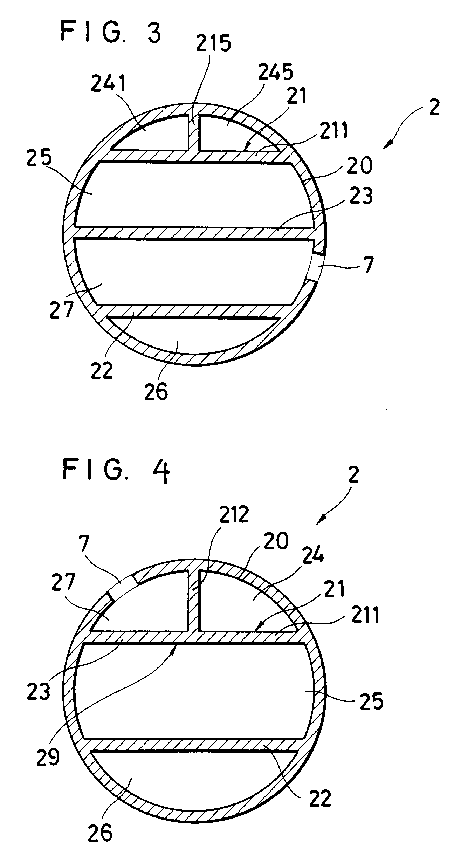

Turning now to FIG. 3, there is illustrated a further embodiment which is a modification of the embodiment of FIGS. 1 and 2.

The embodiment of FIG. 3 is of the five lumen structure obtained by adding a partition segment to the first partition 21 of the FIG. 2 embodiment to divide the thermistor lumen 24 into two thermistor lumens 241 and 245. More particularly, a chordal partition 211 similar to the first partition 21 of the FIG. 2 embodiment is provided and another partition 215 is extended between the tube wall 20 and the partition 211. The other partition 215 defines with the partition 211 sections and the tube wall two thermistor lumens 241 and 245 for receiving thermistors 41 and 45, respectively.

In the FIG. 3 embodiment, both the separated thermistor lumens 241 and 245 belong to the first lumen. The partitions 211 and 215 defining these lumens 241 and 245 with arcuate portions of the tube wall 20 function as the first partition. Both the partitions 211 and 215 are separate from the second partition 22.

FIG. 4 shows a still other embodiment in which two partitions 22 and 29 extend generally parallel to each other and chordally between opposed positions along the tube wall 20. A partition segment 212 extends between the tube wall 20 and the partition 29. A segment 211 of partition 29, partition 212 and a tube wall arcuate portion define a thermistor lumen 24; a segment 23 of partition 29, partition segment 212 and a tube wall arcuate portion define a balloon lumen 27; partitions 29 and 22 and opposed tube wall arcuate portions define an indicator fluid injecting lumen 25; partition 22 and a tube wall arcuate portion define a pressure measuring lumen 26.

In this embodiment, the partition segments 211 and 212 defining the thermistor lumen 24 as the first lumen form the first partition 21, which is separate from the second partition 22 defining the pressure measuring lumen 26 as the second lumen.

It is to be understood that the indicator fluid injecting lumen 25 and the balloon lumen 27 both as the third lumen are defined between the thermistor lumen 24 as the first lumen and the pressure measuring lumen 26 as the second lumen. Namely, the two third lumens are separated by the third partition 23.

In this embodiment, the third partition 23 is connected to the segment 211 of the first partition to form the straight partition 29. Differently stated, the third partition 23 interconnects the tube wall 20 and the first partition 21.

In the design that the first and second partitions 21 and 22 are provided in the tube bore such that they do not interconnect or intersect, a plurality of third lumens can be defined by providing the third partition 23 which is not interconnected or intersected with the first and second partitions 21 and 22 as shown in FIGS. 2 and 3 or by providing the third partition 23 which is interconnected to the first partition 21 and the tube wall 20 as shown in FIG. 4. The benefits of the invention are obtained in either case.

The balloon lumen 27 and the indicator fluid injecting lumen 25 as the third lumen produce a heat insulating function. In such a case, the third partition 23 can interconnect the tube wall 20 and the second partition 22. Alternatively, the third partition 23 can interconnect the first and second partitions 21 and 22. The third partition 23 may comprise a plurality of segments.

Therefore, according to a further aspect of the invention, first and second separate partitions define first and second lumens within the bore of a catheter tube with arcuate portions of the tube wall. At least one third partition is extended between a selected position on the first and second partitions and the tube wall to define at least two third lumens between the first and second partitions.

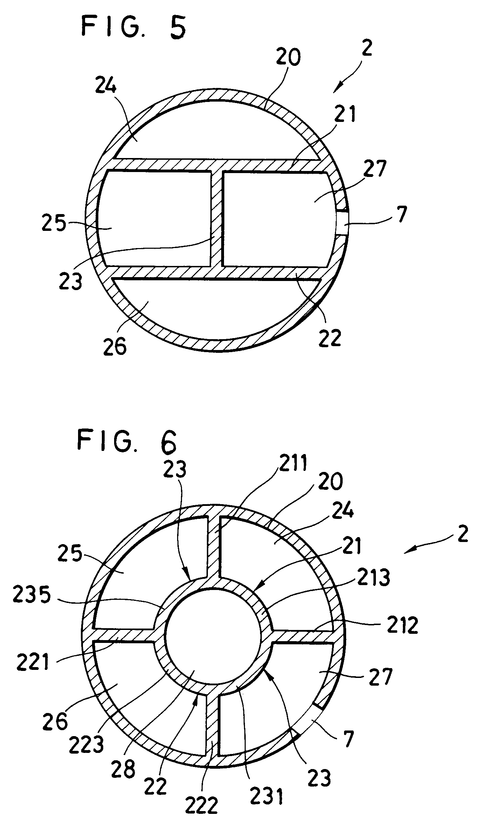

FIG. 5 illustrates one such embodiment in which a catheter is intended to be left in the pulmonary artery. The catheter tube 2 is provided with longitudinally extending, parallel, transversely spaced apart first and second partitions 21 and 22 in the bore to define a first or thermistor lumen 24 and a second or pressure measuring lumen 26. A third partition 23 bridges between the first and second partitions 21 and 22. The third partition 23 intersects the first and second partitions 21 and 22 at right angles and at the center in the figure though not limited thereto. The third partition 23 thus divides the space between the first and second partitions 21 and 22 into a balloon lumen 27 and an indicator fluid injecting lumen 25 (entry of fluid to its distal portion is prevented by a plug as in the previous embodiment), both belonging to the third lumen.

FIG. 6 illustrates another embodiment. The partition means is comprised of a concentric annular section and radial sections radially extending from the annular section to the tube wall. More particularly, a first partition 21 consisting of radial segments 211, 212 and an arcuate segment 213 defines a sector-shaped first or thermistor lumen 24. A second partition 22 consisting of radial segments 221, 222 and an arcuate segment 223 defines a sector-shaped second or pressure measuring lumen 24. The first and second partitions 21 (211, 212, 213) and 22 (221, 222, 223) are connected by arcuate segments 231 and 235 to define three third lumens 25, 27 and 28. Among these, the coaxial sector lumens 25 and 27 disposed along the outer periphery of the bore are an indicator fluid injecting lumen and a balloon lumen, respectively. The central circular lumen 28 is filled with air or a suitable gas for thermal insulation. The central lumen 28 is typically filled with air although it may serve as a self-sustaining means for receiving a guide wire therein for facilitating insertion of the catheter into the body.

It is understood that the third partition segments 231 and 235 interconnect the first and second partitions 21 and 22 in the FIG. 6 embodiment.

FIG. 7 shows a still further embodiment which is a modified version of the FIG. 4 embodiment. The FIG. 7 embodiment is obtained by rotating clockwise the second partition 22 from the position shown in FIG. 4, that is, by moving the connection between the second partition 22 and the tube wall 20 from the position shown in FIG. 4 to the connection between the third partition 23 and the tube wall 20. As a result, the second partition 22 and the first partition segment 211 connected to the third partition 23 form a V shape. Where the indicator fluid injecting lumen 25 opens through the injection port 5, a larger opening is available without reducing the cross-sectional area of the pressure measuring lumen 26, thus offering a reduced resistance against the indicator fluid to be injected and ease of formation of such an opening. The benefits of the invention are also achieved in this embodiment since the first partition 21 consisting of partition segments 211 and 215 is separated from the second partition 22 by the third partition 23.

The material and dimensions of the catheter tube and partitions are not critical to the invention. The catheter tube may be formed of any suitable resin such as polyurethane, polyvinyl chloride, ethylene-vinyl acetate copolymers, polyethylene, and polypropylene. The partitions are preferably formed integral with the tube. The balloon may be formed of latex rubber or silicone rubber. In general, the catheter tube has a wall thickness of about 0.1 to about 0.5 mm and the partitions 21, 22, 23, etc. have a thickness of about 0.05 to 0.5 mm.

Preferably, at least the portion of the catheter tube which is to be left in the blood vessel is coated on the outer surface with an anti-thrombus agent. Such a coating can prevent thrombus formation during continuous cardiac output measurement for a long time. Hydroxyethyl methacrylate and styrene copolymers are preferred anti-thrombus agents.

The construction and operation of the cardiac output measuring catheter other than the partition means forming the present invention are described in US-A-4 841 981.

The cardiac output measuring catheter of the invention enables measurement of a cardiac output while allowing a transfusion fluid to be introduced through a pressure port for pressure measurement at the same time.

## Claims

1. A catheter for measuring a cardiac output, comprising a catheter tube having a wall (20) defining a longitudinal bore therein and having a distal end and a proximal end and an opening (6) near the distal end, a balloon (8) attached to said catheter tube adjacent its distal end, and
partition means (21, 22, 23) in the tube for dividing the bore into a first lumen (24) for installing therein temperature sensing means disposed on a proximal side with respect to said opening, a second lumen (26) in communication with the opening (6) and at least two third lumens,
characterized in that a first partition (21) defining said first lumen (24) is disposed so as not to intersect a second partition (22) defining said second lumen (26), whereby said first and second lumens are separated by an injecting lumen (25) having an injection port (5) for allowing an indicator fluid to pass therethrough and exit through the injection port, said injection port being located on a proximal side with respect to the temperature sensing means (41, 45) and by a balloon lumen (27) in communication with said balloon (8).

2. The catheter of claim 1 wherein said partition means includes a third partition (23) for defining said two third lumens.

3. The catheter of claim 2 wherein said third partition (23) extends between opposed positions along the tube wall (20).

4. The catheter of claim 2 wherein said third partition (23) extends between the first (21) and second (22) partitions.

5. The catheter of any preceding claim wherein said temperature sensing means includes a thermistor for thermodilution measurement of a cardiac output and a heating thermistor for measurement of blood flow velocity.

6. The catheter of any preceding claim wherein said second lumen is in fluid communication with the exterior through said opening for allowing measurement of the external pressure.

## Patentansprüche

1. Katheter zur Messung des Herzleistung, umfassend
- ein Katheterrohr, das eine Wand (20), die eine Längsbohrung darin umgrenzt und ein distales Ende und ein proximales Ende und eine Öffnung (6) nahe dem distalen Ende aufweist, ein Ballon (8) aufweist, der an dem Katheterrohr in der Nähe von dessen distalem Ende angebracht ist, und
- Trenneinrichtungen (21, 22, 23) im Rohr zur Aufteilung der Bohrung in ein erstes Lumen (24) zur Anbringung einer Temperaturfühleinrichtung darin, die auf einer proximalen Seite in bezug auf die Öffnung angeordnet ist, ein zweites Lumen (26) in Verbindung mit der Öffnung (6) und mindestens zwei dritte Lumen,
dadurch **gekennzeichnet,** daß eine erste, das erste Lumen (24) umgrenzende Trennwand (21), so angeordnet ist, daß sie eine zweite, das zweite Lumen (26) umgrenzende Trennwand (22) nicht schneidet, wodurch die ersten und zweiten Lumen durch ein Injektionslumen (25) getrennt sind, das eine Injektionsöffnung (5) aufweist, um es zu gestatten, daß eine Indikatorflüssigkeit hindurchfließt und durch die Injektionsöffnung austritt, wobei sich die Injektionsöffnung in bezug auf die Temperaturfühleinrichtung (41, 45) an einer proximalen Seite und durch ein Ballonlumen (27) in Verbindung mit dem Ballon (8) befindet.

2. Katheter nach Anspruch 1, bei dem die Trenneinrichtung eine dritte Trennwand (23) zur Umgrenzung der zwei dritten Lumen umfaßt.

3. Katheter nach Anspruch 2, bei dem sich die dritte Trennwand (23) zwischen gegenüberliegenden Positionen entlang der Rohrwand (20) erstreckt.

4. Katheter nach Anspruch 2, bei dem sich die dritte Trennwand (23) zwischen der ersten (21) und zweiten (22) Trennwand erstreckt.

5. Katheter nach einem beliebigen vorhergehendem Anspruch, bei dem die Temperaturfühleinrichtung einen Thermistor zur Thermodilutionsmessung einer Herzleistung und einen Heiz-Thermistor zur Messung der Blutströmungsgeschwindigkeit umfaßt.

6. Katheter nach einem beliebigen vorhergehenden Anspruch, bei dem das zweite Lumen durch die Öffnung in Flüßigkeitskontakt mit der Außenseite steht, um die Messung des Außendrucks zu gestatten.

## Revendications

1. Cathéter pour mesurer le débit cardiaque, qui comprend:
- un tube de cathéter ayant une paroi (20) qui y définit un passage central longitudinal et ayant une extrémité distale et une extrémité proximale ainsi qu'une ouverture (6) près de l'extrémité distale, un ballonnet (8) étant fixé audit tube de cathéter à proximité de son extrémité distale, et
- des moyens formant cloisons (21, 22, 23) dans le tube pour diviser le passage central en une première lumière (24) dans laquelle sera placé un moyen de détection de température disposé sur le côté proximal par rapport à ladite ouverture, une seconde lumière (26) en communication avec l'ouverture (6) et au moins deux troisièmes lumières,
caractérisé en ce que la première cloison (21) définissant ladite première lumière (24) est placée de manière à ne pas couper une seconde cloison (22) définissant ladite seconde lumière (26), ce qui fait que lesdites première et seconde lumières sont séparées par une lumière d'injection (25), comportant une embouchure d'injection (5) qui va permettre le passage d'un fluide indicateur et sa sortie par l'embouchure d'injection, ladite embouchure d'injection étant placée sur le côté proximal par rapport aux moyens (41, 45) de détection de la température, et par une lumière de ballonnet (27) en communication avec ledit ballonnet (8).

2. Cathéter selon la revendication 1, dans lequel ledit moyen formant cloisons comprend une troisième cloison (23) pour définir lesdites deux troisièmes lumières.

3. Cathéter selon la revendication 2, dans lequel ladite troisième cloison (23) s'étend entre des positions opposées le long de la paroi (20) du tube.

4. Cathéter selon la revendication 2, dans lequel ladite troisième cloison (23) s'étend entre les première (21) et seconde (22) cloisons.

5. Cathéter selon l'une quelconque des précédentes revendications, dans lequel lesdits moyens de détection de la température comprennent une thermistance pour la mesure par thermodilution du débit cardiaque et une thermistance chauffante pour mesurer la vitesse du flux sanguin.

6. Cathéter selon l'une quelconque des précédentes revendications, dans lequel ladite seconde lumière est en communication assurant le passage des fluides avec l'extérieur grâce à ladite ouverture afin de permettre une mesure de la pression externe.
